# EUROPÄISCHE PATENTSCHRIFT

(11) **EP 3 412 325 B1**
(45) Veröffentlichungstag und Bekanntmachung des Hinweises auf die Patenterteilung: **25.09.2019**
(21) Anmeldenummer: 17175093.8
(22) Anmeldetag: 08.06.2017
(51) Int. Cl.: A61M 15/00, A61M 15/08, B65D 83/14, A61M 11/00

(54) **AUSTRAGKOPF FÜR DIE NASALE APPLIKATION VON FLÜSSIGKEIT AUS EINEM DRUCKSPEICHER**
APPLICATOR HEAD FOR NASAL APPLICATION OF FLUID FROM A PRESSURE ACCUMULATOR
TÊTE DISTRIBUTRICE POUR L'APPLICATION NASALE DE LIQUIDE PROVENANT D'UN ACCUMULATEUR DE PRESSION

(43) Veröffentlichungstag der Anmeldung: 12.12.2018
(73) Patentinhaber: Aptar Radolfzell GmbH, 78315 Radolfzell (DE)
(72) Erfinder: Greiner-Perth, Jürgen, 78244 Gottmadingen (DE); Schmid, Felix, 78337 Öhningen (DE)
(74) Vertreter: Patentanwaltskanzlei Cartagena

(56) Entgegenhaltungen:
- WO-A1-2017/021878
- DE-A1-102012 200 545
- DE-T2- 69 914 204

## Beschreibung

### ANWENDUNGSGEBIET UND STAND DER TECHNIK

Die Erfindung betrifft einen Austragkopf für die nasale Applikation von pharmazeutischen Flüssigkeit aus einem Druckspeicher, welcher über ein Auslassventil mit einem Ventilstutzen verfügt, der gegen eine Federkraft zum Öffnen des Auslassventils kraftbeaufschlagbar ist. Die Erfindung betrifft weiterhin auch einen Spender mit einem solchen Austragkopf.

Ähnliche Austragköpfe und Spender sind beispielsweise aus der DE 102012200545 A1 bekannt. Die in dieser Schrift offenbarten Austragköpfe verfügen über ein Außengehäuse, welches einerseits die Basis zur Anbindung an einem Druckspeicher beinhaltet und andererseits die Außenschale eines Nasalapplikators mitsamt Austragöffnung bildet. In den länglichen Nasalapplikator ist ein Innenbauteil eingeschoben, welches gemeinsam mit der Außenschale der Fluidführung von Flüssigkeit zur Austragöffnungdient.

Auch aus dem US Design Patent mit der Publikationsnummer US D 659,531 S ist eine ähnliche Gestaltung bekannt.

Gattungsgemäße Austragköpfe werden für den Vertrieb von Medikamenten mit vergleichsweise geringem Verkaufspreis verwendet. Es ist daher erforderlich, dass die Herstellung der Austragköpfe preisgünstig möglich ist. Die diesbezügliche vorteilhafte einstückige Gestaltung des Außengehäuses mit Basis und Nasalapplikator ist zur Erzielung geringer Kosten gut geeignet. Allerdings sind auch Nachteile gegeben. Das Zuführen des flüssigen Kunststoffes im Rahmen des Spritzguß-Vorgangs erfolgt üblicherweise im Bereich der Basis. Die Austragöffnung ist daher vergleichsweise weit vom Zuführpunkt entfernt, was zu einer mitunter nicht zufriedenstellenden Fertigungsgenauigkeit an der Austragöffnung und an Fluidleitflächen stromaufwärts der Austragöffnung führt. Dies ist von Nachteil, da hierdurch das Austragverhalten der Flüssigkeit von Spender zu Spender variieren kann und nicht mehr dem beabsichtigten Austragverhalten entspricht.

Aufgabe der Erfindung ist es daher, einen gattungsgemäßen Spender dahingehend weiterzubilden, dass eine hohe Fertigungsgenauigkeit im Bereich der Austragöffnung erzielt wird, ohne die Komplexität der Fertigung relevant zu erhöhen.

Die der Erfindung zugrunde liegende Aufgabe wird durch einen Austragkopf gelöst, der in Übereinstimmung mit gattungsbildenden Austragköpfen über eine Basis, eine demgegenüber bewegliche Betätigungsfläche sowie einen Nasalapplikator verfügt. Die Basis weist eine daran vorgesehene Kopplungseinrichtung auf, mittels derer der Austragkopf am Druckspeicher befestigbar ist.

Die Betätigungsfläche ist gegenüber der Basis zum Zwecke der Betätigung manuell verlagerbar. Ein an der Betätigungsfläche vorgesehener Stößel in Art eines Hohlrohrs wird hierdurch mit verlagert, so dass hierdurch der Ventilstutzen des Ventils des Druckspeichers geöffnet werden kann. Vorzugsweise ist die Betätigungsfläche mit einer Strukturierung, beispielsweise einer Riffelung versehen.

Der Nasalapplikator erstreckt sich von der Betätigungsfläche weg nach außen. An seinem distalen Ende ist eine Austragöffnung vorgesehen, die durch einen innenliegenden Applikatorkanal des Nasalapplikators hindurch mit dem Hohlrohrfluidkommunizierend verbunden ist.

Der Nasalapplikator verfügt erfindungsgemäß über ein Innenbauteil, welches einstückig mit der Betätigungsfläche verbunden ist. Weiterhin verfügt er über ein Außenbauteil, welches als vom Innenbauteil getrenntes Bauteil ausgebildet ist und das Innenbauteil umgebend an diesem angebracht ist. Die Austragöffnung ist als Durchbrechung des Außenbauteils vorgesehen. Der zur Austragöffnung führende Applikatorkanal ist gemeinsam durch das Außenbauteil und das Innenbauteil begrenzt.

Bei einem erfindungsgemäßen Nasalapplikator ist also vorgesehen, dass die Austragöffnung durch ein Bauteil gebildet wird, welches zumindest vom Innenbauteil und der damit einstückig ausgebildeten Betätigungsfläche getrennt ist. Die Austragöffnung ist damit Teil eines insgesamt kleineren Bauteils als bei den Gestaltungen gemäß dem Stand der Technik. Dies gestattet es, die Austragöffnung in höherer Präzision und/oder bei höheren Taktzahlen zu fertigen.

Unter der Austragöffnung im Sinne der Erfindung ist das Ende des Applikatorkanals zu verstehen, also der Übergangspunkt, an dem die druckbeaufschlagte Flüssigkeit in eine äußere Umgebung unter Umgebungsdruck abgegeben wird und dort je nach Art des Austritts durch die Austragöffnung als freier Jet oder als Sprühstrahl vorliegt. Die Austragöffnung weist vorzugsweise eine minimale Querschnittsfläche von weniger als 4 mm² auf, insbesondere von weniger als 2 mm².

Neben dem Vorteil der besseren Herstellbarkeit ist die Ausbildung des Außenbauteils als von der Basis separates Bauteil auch deshalb von Vorteil, da hierdurch eine Austauschbarkeit des Außenbauteils erzielt werden kann, die aus Hygienegründen von Vorteil ist. Insbesondere die Austragöffnung, an der nach der Nutzung Flüssigkeit verbleiben kann, kann schnell verkeimen. Durch einen Austausch des Außenbauteils werden alle oder zumindest besonders gefährdete Bereiche ausgetauscht. Auch die Anpassung des Austragkopfes an eine bestimmte Zielgruppe ist durch das separate Außenbauteil möglich. So kann insbesondere eine erwachsenengerechte bzw. kindgerechte Gestaltung durch die Wahl des passenden Außenbauteils erzielt werden. Dabei ist sowohl möglich, dass bereits herstellerseitig eine zielgruppenspezifische Anpassung erfolgt, als auch, dass mehrere Außenbauteile an den Endkunden mitgeliefert werden, so dass er selbst die Anpassung einmalig oder fallweise vornehmen kann.

Der Nasalapplikator wird bestimmungsgemäß in ein Nasenloch des Nutzers eingeführt. Zu diesem Zweck handelt es sich um einen länglichen, schlanken Applikator, der vorzugsweise eine sich zum distalen Ende und der Austragöffnung verjüngende Formgebung aufweist. Vorzugsweise ragt der Nasalapplikator ausgehend vom Niveau der Betätigungsfläche mindestens 20mm hervor. Seine frei von der Basis bzw. der Betätigungsfläche hervorstehende Länge ist vorzugsweise mindestens um Faktor 2, insbesondere vorzugsweise mindestens um Faktor 2,5, größer als der maximale Durchmesser des Nasalapplikators.

Die Basis des Austragkopfes ist jener Teil, der bestimmungsgemäß am Druckspeicher angekoppelt wird. Hierfür ist die Kopplungseinrichtung vorgesehen, die insbesondere vorzugsweise über angeformte Rastkanten auf dem Druckspeicher aufgeschnappt wird. Die Basis verfügt hierfür vorzugsweise über eine ringartige Struktur mit nach innen weisenden Rastnasen. Bei bevorzugt mit einem erfindungsgemäßen Austragkopf verwendeten Druckspeichern ist eine Crimp-Verbindung zwischen einem Deckel und einem Mantel vorgesehen, welche vorzugsweise zum Aufschnappen der Rastnasen verwendet wird.

Das Innenbauteil und das Außenbauteil bilden vorzugsweise gemeinsam eine der Austragöffnung vorgelagerte Wirbelkammer. Unter einer Wirbelkammer wird dabei eine der Austragöffnung vorgelagerte Kammer verstanden, in die die Flüssigkeit zumindest teilweise in tangentialer Richtung oder zumindest gegenüber einer Radialrichtung angewinkelter Richtung einströmt.

Eine solche Wirbelkammer weist vorzugsweise mindestens eine Leitfläche zur Erzeugung eines Dralls auf, die gegenüber einer Radialrichtung schräggestellt ist. Diese mindestens eine Leitfläche ist vorzugsweise an einer Innenseite des Außenbauteils vorgesehen, so dass wie auch bei der Austragöffnung eine große Fertigungspräzision beim Spritzgießen erzielt werden kann. Die Leitfläche kann insbesondere als Teil eines in die Wirbelkammer mündenden Zuströmkanals vorgesehen sein. Insbesondere vorzugsweise ist eine Mehrzahl solcher Zuströmkanäle vorgesehen.

Grundsätzlich ist eine Ausgestaltung des Austragkopfes möglich, bei der das Außenbauteil und das Innenbauteil im montierten Zustand ortsfest zueinander sind.

Besonders von Vorteil ist die erfindungsgemäße Gestaltung jedoch bei einer Ausgestaltung, bei der das Außenbauteil derart am Innenbauteil angebracht ist, dass das Außenbauteil im angebrachten Zustand gegenüber dem Innenbauteil relativbeweglich ist.

Das Innenbauteil und das Außenbauteil gestatten es demnach, dass das Außenbauteil gegenüber dem Innenbauteil bewegt wird. Hierdurch kann eine Anpassung des Austragkopfes und insbesondere des Nasalapplikators an verschiedene Situationen erzielt werden, wie im Weiteren noch erläutert wird.

Die erfindungsgemäße Ausgestaltung mit einem auf das Innenbauteil aufgesetzten Außenbauteil gestattet eine solche Ausgestaltung mit beweglichem Außenbauteil in vorteilhafter Gestaltung, da die Relativverlagerung durch den Benutzer in einfacher Weise erfolgen kann, indem das Außenbauteil unmittelbar und manuell gegenüber der Basis kraftbeaufschlagt wird.

Die Bewegung des Innenbauteils und des Außenbauteils erfolgt vorzugsweise zwischen zwei Endlagen, die einer fortgesetzten Bewegung im Wege stehen oder diese zumindest erschweren. Allerdings kann es zweckmäßig sein, dass eine Relativbewegung in einer Richtung mit erhöhter Kraft über die zunächst die Relativbewegung unterbrechende Endlage hinaus erfolgen kann, so dass bei geringem Kraftaufwand eine Verlagerung zwischen den Endlagen möglich ist und mit einer demgegenüber größeren Kraftbeaufschlagung das Innenbauteil und das Außenbauteil zerstörungsfrei über diese Endlage hinaus bewegt werden können, so dass das Innenbauteil und das Außenbauteil hierdurch voneinander getrennt werden können, um bspw. aus hygienischen Gründen das Außenbauteil gegen ein anderes auszutauschen.

Die Verlagerbarkeit des Außenbauteils und des Innenbauteils gegeneinander kann insbesondere dem Zweck dienen, die Charakteristik des Flüssigkeitsaustrags durch die Austragöffnung hindurch zu verändern.

Das Innenbauteil und das Außenbauteil sind hierfür vorzugsweise derart ausgebildet, dass durch eine Relativverlagerung des Innenbauteils und des Außenbauteils der Applikatorkanal und/oder die Wirbelkammer geometrisch veränderlich ist.

Insbesondere können das Innenbauteil und das Außenbauteil derart ausgebildet sein, dass durch eine Relativverlagerung der Applikatorkanal bezüglich seines lichten Querschnitts veränderlich ist. Je nachdem, in welchem Bereich des Applikatorkanals die Veränderung erfolgt, lassen sich hierdurch verschiedene Wirkungen erzielen.

Wenn der Applikatorkanal weit vor der Austragöffnung durch einer Relativverlagerung verbreitert oder verjüngt wird, führt dies zu einer Drosselwirkung, mittels derer in zwei unterschiedlichen Stellungen des Außenbauteils relativ zum Innenbauteil bei gleichem Einströmdruck unterschiedliche Flüssigkeitsströme durch die Austragöffnung ausgegeben werden. Es kann also beispielsweise eine erwachsenengerechte und kindgerechte Dosierung eingestellt werden, ohne dass diese Dosierung durch längere und kürzere Betätigung erfolgen muss.

Wird der Applikatorkanal im Bereich der ihn beendenden Wirbelkammer bezüglich der lichten Weite verändert, so lässt sich die Charakteristik der durch die Austragöffnung ausströmenden Flüssigkeit beeinflussen. Insbesondere kann hierdurch erzielt werden, dass bei gleichem Einströmdruck Flüssigkeit in Abhängigkeit der Relativstellung mit einem unterschiedlichem Maß von Tröpfchenbildung oder unterschiedlicher Tröpfchengröße ausgegeben.

Dies wird insbesondere dadurch erzielt, dass die Wirkung einer Drall bewirkenden Geometrie dadurch erhöht oder abgeschwächt wird, dass abhängig von der Relativstellung ein unterschiedlicher Anteil der Flüssigkeit durch Drall bewirkende Kanäle geleitet wird, während der verbleibende Anteil an diesen Drall bewirkenden Kanälen vorbei direkt zur Austragöffnung geleitet wird.

So ist beispielsweise eine Gestaltung möglich, bei der zwischen einem unzerstäubten Flüssigkeitsstrahl, auch Jet genannt, und einer Zerstäubung umgeschaltet werden kann. Zusätzlich oder alternativ kann auch ein vollständiges Verschließen des Applikatorkanals möglich sein, so dass eine Art Transportsicherung geschaffen wird.

Die genannte Relativbeweglichkeit zwischen Außenbauteil und Innenbauteil kann auf verschiedene Art und Weise realisiert sein. So kann vorgesehen sein, dass das Außenbauteil gegenüber dem Innenbauteil rein linear oder rein rotativ beweglich ist. Auch möglich ist eine Kombination, insbesondere bei der das Außenbauteil gegenüber dem Innenbauteil überlagert linear und rotativ beweglich ist.

Bei einer rein rotativen Verlagerbarkeit ist vorzugsweise vorgesehen, dass der Applikatorkanal insgesamt eine von der Rotationssymmetrie abweichende Formgebung aufweist, so dass die Formgebung des Applikatorkanals durch ein Verdrehen beeinflussbar ist.

Bei einer rein oder kombiniert linearen Beweglichkeit wird auch die Austragöffnung selbst durch die Verlagerung axial gegenüber dem Innenbauteil verlagert. Dies eignet sich insbesondere, um durch diese axiale Bewegung den Applikatorkanal unmittelbar vor der Wirbelkammer oder vor der Austragöffnung vollständig zu unterbrechen und/oder in oben genannter Weise zu beeinflussen, welcher Anteil der Flüssigkeit beim Einströmen in eine Wirbelkammer drallbeaufschlagt werden soll.

Vorzugsweise ist in einem Übergangsbereich zwischen dem Innenbauteil und der Betätigungsfläche eine Vertiefung vorgesehen. Ein proximaler Rand des Außenbauteils taucht in mindestens einer Endstellung des Außenbauteils gegenüber dem Innenbauteil in diese Vertiefung ein.

Die genannte Vertiefung ist vorzugsweise nutartig ausgestaltet. Ein proximales, der Betätigungsfläche zugewandtes und der Austragöffnung abgewandtes Ende des Außenbauteils taucht zumindest phasenweise in diese Vertiefung ein. Dies hat ästhetische Vorteile und ist auch funktional von Vorteil, da ein versehentliches Untergreifen und Hängenbleiben am proximalen Ende der Außenhülse erschwert wird. Auch die Gefahr des Eindringens von Schmutz wird verringert.

Vorzugsweise sind das Innenbauteil und das Außenbauteil zwischen zwei Endstellungen gegeneinander beweglich, wobei der proximale Rand des Außenbauteils zumindest abschnittsweise und vorzugsweise umlaufend in beiden Endstellungen innerhalb der Vertiefung angeordnet ist. Bei einer solchen Gestaltung ist das proximale Ende zumindest abschnittsweise unabhängig von der Relativstellung von Außenbauteil und Innenbauteil stets in derVertiefung angeordnet. Besonders vorteilhaft ist es, wenn die Nut ausreichend tief ist, dass das proximale Ende ungeachtet der Relativstellung des Außenbauteils zum Innenbauteil stets umlaufend in der Nut verbleibt.

Eine bevorzugte Ausgestaltung sieht vor, dass das Innenbauteil über einen ersten Hülsenabschnitt verfügt und das Außenbauteil über einen zweiten Hülsenabschnitt verfügt. Eine Außenseite des ersten Hülsenabschnitts weist dabei Fixierung- und/oder Führungsmittel auf, mittels derer das Außenbauteil am Innenbauteil geführt und/oder befestigt ist. Eine Innenseite des ersten Hülsenabschnitts weist eine Dichtfläche auf, an der der zweite Hülsenabschnitt derart dichtend anliegt, dass ein gemeinsamer Innenraum beider Hülsenabschnitte einen Abschnitt des Applikatorkanals bildet, mittels dessen die Austragöffnung mit dem als Hohlrohr ausgebildeten Stößel verbunden ist.

Der beschriebene Aufbau mit einem ersten Hülsenabschnitt als Teil des Innenbauteils ist von Vorteil, da dieser Hülsenabschnitt eine Doppelfunktion erfüllt. Durch Strukturen an seiner Außenseite wirkt er mit dem Außenbauteil dahingehend zusammen, dass dieses hier eingerastet ist oder durch Anschläge bewegungslimitiert ist. Auch ein Außengewinde zur Zusammenwirkung mit einem Innengewinde am Außenbauteil kann an der Außenseite des ersten Hülsenabschnittes vorgesehen sein. Die zweite Funktion liegt in der innenseitigen Dichtfläche, die gemeinsam mit dem zweiten Hülsenabschnitt des Außenbauteils eine Abdichtung des Applikatorkanals nach außen schafft, vorzugsweise in Art eines teleskopierbaren Kanalabschnittes.

Insbesondere von Vorteil ist es, wenn das Innenbauteil einen Hülsenabschnitt zum Zwecke der Abdichtung gegenüber dem Außenbauteil und/oder zum Zwecke der Kopplung des Außenbauteils am Innenbauteil umfasst sowie einen zentrischen Stift, dessen distales Ende mit der Austragöffnung zur Beeinflussung des Austrags zusammenwirkt, wobei ein durch das Hohlrohr gebildeter Kanal in einen Zwischenraum zwischen dem zentrischen Stift und dem Hülsenabschnitt mündet.

Das Innenbauteil ist bei einer solchen Gestaltung also gleichsam zweigeteilt. Der umlaufende Hülsenabschnitt wirkt mit dem Außenbauteil abdichtend zusammen. Der zentrische Stift kann insbesondere der Bildung der Wirbelkammer dienen oder in anderer Art und Weise mit dem Außenbauteil, insbesondere mit dessen Stirnseite mit Austragöffnung, zusammenwirken, so dass hierdurch die Flüssigkeitsführung und/oder der Flüssigkeitsaustrag beeinflusst oder auch fallweise unterbunden werden kann.

Eine erfindungsgemäße Ausgestaltung zeichnet sich durch einen recht einfachen Aufbau auf. Insbesondere von Vorteil ist es, wenn die Betätigungsfläche und die Basis sowie die daran vorgesehene Kopplungseinrichtung des Austragkopfes als einstückiges Bauteil ausgebildet sind. Die Betätigungsfläche und der als Hohlrohr ausgebildete Stößel sind ebenfalls vorzugsweise als einstückiges Bauteil ausgebildet.

Durch eine kombinierte Ausgestaltung dessen lässt sich ein Austragkopf erzielen, der aus nur zwei Bauteilen besteht, nämlich einem ersten Bauteil, umfassend die Basis, die Betätigungshandhabe mit Betätigungsfläche sowie das Innenbauteil des Nasalapplikators, wobei mindestens die Betätigungshandhabe durch Materialverformbarkeit gegenüber der Basis verlagerbar ist, und einem zweiten Bauteil, welches das Außenbauteil des Nasalapplikator bildet. Es ist somit eine sehr kostengünstige Bauweise möglich.

Das Außenbauteil und die Betätigungsfläche weisen vorzugsweise unterschiedliche Farbgebungen und/oder unterschiedliche Materialen auf. Die Zweifarbigkeit von Basis und Außenbauteil kann insbesondere zum Zwecke der ästhetischen Verbesserung genutzt werden, jedoch auch, um den Austragkopf an eine unternehmensspezifische typische Farbgebung anzupassen. Die Verwendung verschiedener Materialien gestattet es darüber hinaus, jeweils ideal angepasste Materialien für das Außenbauteil und die Basis mitsamt Betätigungshandhabe zu wählen. So könnte das Außenbauteil beispielsweise aus einem Material gefertigt sein, welches durch Beschichtung oder inhärente Materialeigenschaften Bakterienwachstum verhindert oder welches in Kontakt mit der Haut des Nutzers als haptisch angenehm empfunden wird, während das Hauptbauteil mit Basis und Betätigungshandhabe aus einem anderen Material bestehen kann.

Vorzugsweise ist die Betätigungsfläche schwenkbeweglich an der Basis angebracht, insbesondere vorzugsweise durch eine einstückig die Basis und die Betätigungsfläche verbindende Kunststoffbrücke, die als Schwenkgelenk agiert. Insbesondere von Vorteil ist es weiterhin, wenn auf einer der Kunststoffbrücke gegenüberliegenden Seite der Betätigungsfläche im Lieferzustand eine Sicherungsbrücke vorgesehen ist, die die Betätigungsfläche und die Basis einstückig verbindet und bei Erstbetätigung bestimmungsgemäß bricht. Die genannte Einstückige Gestaltung mit verformbarer Kunststoffbrücke als Gelenk ist im Sinne eines Aufbaus aus wenig Einzelteilen von Vorteil. Die Betätigungsfläche ist abseits der Kunststoffbrücke gegenüber der Basis durch einen Schlitz freigeschnitten, so dass sie beweglich ist. Durch die Sicherungsbrücke wird erreicht, dass es vor Inbetriebnahme nicht zu einem ungewollten Austrag von Flüssigkeit kommt, da die Inbetriebnahme zum Zwecke des Brechens dieser Sicherungsbrücke eine stärkere Kraftbeaufschlagung erfordert.

Eine durch die Austragrichtung der Austragöffnung und/oder durch eine die Rotationsmittelachse des Außenbauteils gegenüber dem Innenbauteil definierte Achse schließt vorzugsweise mit einer Aufsetzrichtung des Austragkopfs auf einen Druckspeicher einen Winkel α zwischen 5° und 85° ein, vorzugsweise einen Winkel zwischen 10° und 50°.

Diese Anwinkelung ist bei gattungsgemäßen Austragköpfen üblich. Sie gestattet eine vorteilhafte Verwendung, bei der der Druckspeicher, dessen Haupterstreckungsrichtung vorzugsweise mit der Aufsetzrichtung übereinstimmt, leicht angewinkelt gehalten werden kann und der Nasalapplikator dann in passendem Winkel in ein Nasenloch eingeschoben werden kann. Bei einem erfindungsgemäßen Austragkopf ist vorzugsweise weiterhin vorgesehen, dass die Betätigungsfläche und der Nasalapplikator nebeneinander angeordnet sind, so dass eine asymmetrische Formgebung des Austragkopfes gegeben ist.

Die Erfindung betrifft weiterhin auch einen Spender zum Austrag pharmazeutischer Flüssigkeiten mit einem Austragkopf der vorbeschriebenen Art. Dieser Austragkopf ist an einem Druckspeicher des Spenders befestigt, in welchem pharmazeutische Flüssigkeit unter Druck gelagert ist und der seinerseits über ein Auslassventil verfügt. Das Auslassventil verfügt über einen Ventilstutzen, der gegen eine Federkraft zum Öffnen des Ventils mittels des Stößels des Austragkopfes kraftbeaufschlagbar ist.

Der Druckspeicher wird vorzugsweise durch eine bezüglich der Außenform rotationssymmetrische Mantelwandung begrenzt, die einstückig oder mehrstückig mit einem vorzugsweise bombierten Boden ausgebildet ist. Das integrierte Ventil ist vorzugsweise in ein Deckelteil integriert, welches mittels einer Crimp-Verbindung mit der Mantelfläche verbunden wird. Der Verbindungsbereich zwischen Mantelfläche und Deckelteil bildet vorzugsweise jene Rastkante, im Bereich derer der Austragkopf auf den Druckspeicher aufgerastet ist.

Einem Druckspeicher ist es zu eigen, dass die darin gespeicherte Flüssigkeit stets unter Druck steht, so dass der Druck nicht für einen Austragvorgang erzeugt werden muss. Für die Ausübung des Drucks sind verschiedene Möglichkeiten gegeben, so die Druckbeaufschlagung per Treibgas oder mittels komprimierter Luft im Flüssigkeitsspeicher oder insbesondere in einem Zwischenbereich zwischen einem Beutel im Druckspeicher und der Mantelwandung des Druckspeichers.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A und 1B zeigen einen erfindungsgemäßen Spender in geschnittener und ungeschnittener Gesamtdarstellung.
Fig. 2 zeigt die Innenformgebung einer Außenhülse eines Nasalapplikators des Austragkopfes eines solchen Spenders.
In den Fig. 3 sowie 3A und 3B ist ein erstes Ausführungsbeispiel des Austragkopfes im Detail dargestellt.
In den Fig. 4 sowie 4A und 4B ist ein zweites Ausführungsbeispiel des Austragkopfes im Detail dargestellt.

### DETAILLIERTE BESCHREIBUNG DERAUSFÜHRUNGSBEISPIELE

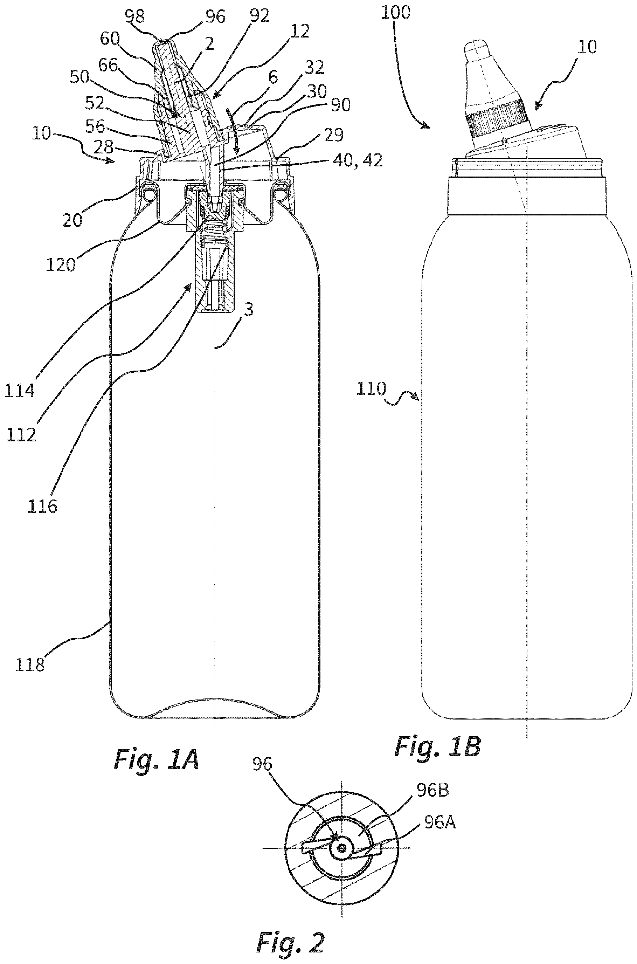

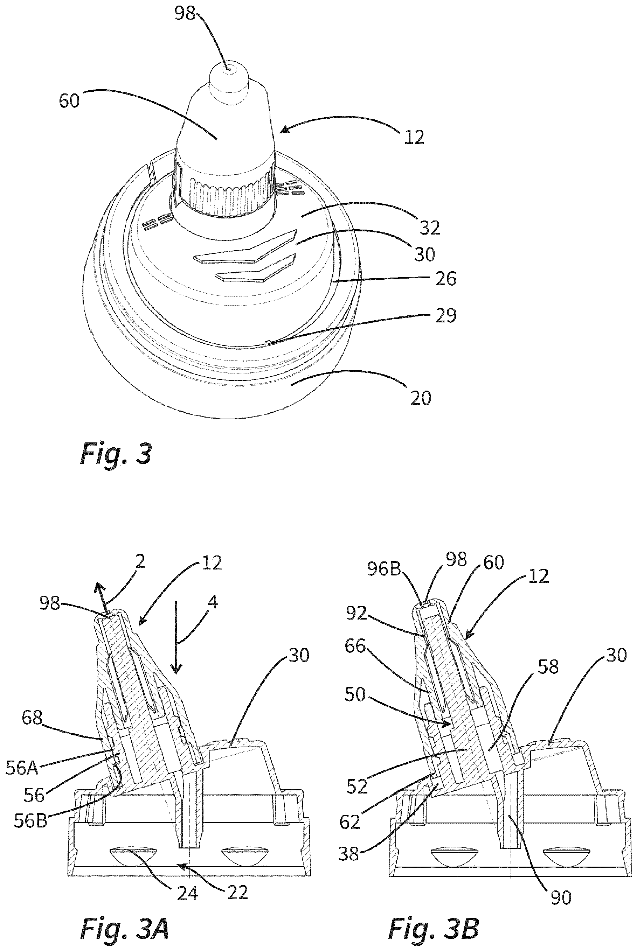

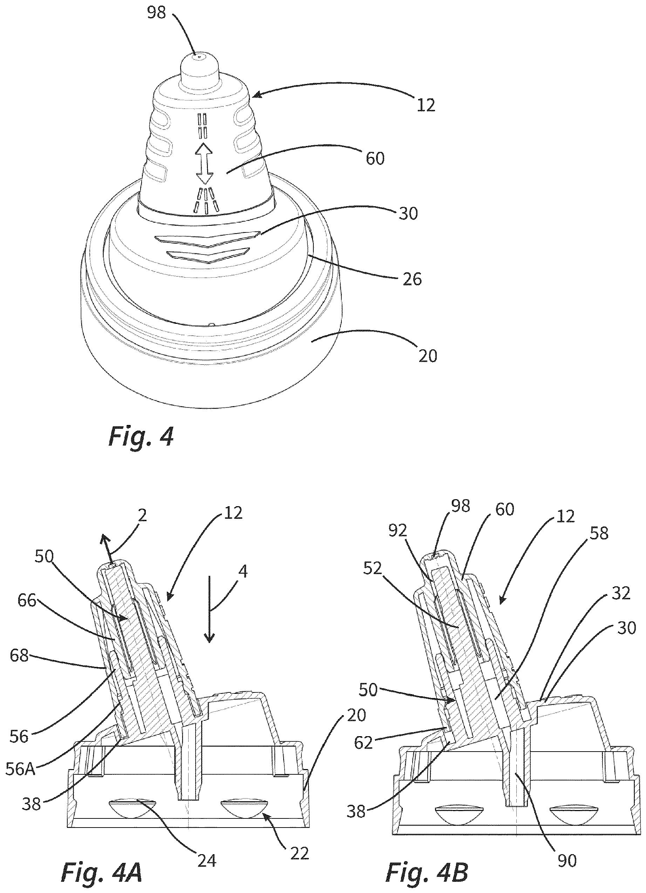

Die Fig. 1A und 1B zeigen eine Gesamtdarstellung eines erfindungsgemäßen Spenders 100 in geschnittener und ungeschnittener Darstellung. Dabei gelten die hierzu nachfolgend erläuterten Zusammenhänge für verschiedene Ausgestaltungen des Austragkopfes 10 dieses Spenders 100. Details zu zwei möglichen Ausgestaltungen des Austragkopfes 10 werden anschließend anhand der Fig. 3 und 4 erläutert.

Ein erfindungsgemäßer Spender 100 gemäß den vorliegenden Ausführungsbeispielen verfügt über einen Druckspeicher 110, dessen Außenflächen durch einen metallischen Korpus 118 und einen Deckel 120 gebildet werden. Im Deckel 120 ist ein Auslassventil 112 befestigt, welches durch Niederdrücken eines Ventilstutzens 114 gegen die Kraft einer Ventitfeder 116 geöffnet werden kann, so dass die Flüssigkeit in den Austragkopf 10 einströmt.

Der erfindungsgemäße Spender 100 verfügt am Austragkopf 10 über einen schlanken, länglichen Nasalapplikator 12, dessen Haupterstreckungsrichtung gegenüber der Haupterstreckungsrichtung 3 des Druckspeichers 110 angestellt ist. Der Nasalapplikator 12 ist zum Einschieben in ein Nasenloch eines Benutzers vorgesehen.

Der Austragkopf 10 ist im Bereich einerCrimp-Verbindungzwischen dem Korpus 118 und dem Deckel 120 auf den Druckspeicher 110 aufgeschnappt, wobei hierfür eine im Weiteren noch erläuterte Kopplungseinrichtung 22 an einer Basis 20 des Austragkopfes vorgesehen ist.

Die Basis 20 des Austragkopfes 10 ist mit der überwiegenden Zahl der funktionalen Elemente des Austragkopfes 10 einstückig verbunden.

So ist über eine Kunststoffbrücke 28 eine Betätigungshandhabe 30 mit Betätigungsfläche 32 einstückig an der Basis 20 angeformt. An dieser Betätigungshandhabe 30 ist ein als Hohlrohr 42 ausgestalteter Stößel 40 einstückig angeformt, der in das Auslassventil 112 des Druckspeichers 110 zum Zwecke der Betätigung und zum Zwecke des Einströmens von Flüssigkeit eingeschoben ist.

Darüber hinaus ist auch ein Innenbauteil 50 des Nasalapplikators 12 des Austragkopfes 10 einstückig mit der Betätigungshandhabe 30 ausgebildet. Dieses Innenbauteil 50 umfasst einen außenliegenden Hülsenabschnitt 56 und einen innenliegenden Stift 52.

Das einzige von diesem Verbundbauteil getrennte Bauteil des Austragkopfes 10 ist das Außenbauteil 60 des Nasalapplikators 12, an dessen Stirnseite eine Stirnwandung durch eine Austragöffnung 98 durchbrochen ist. Das Außenbauteil 60 ist auf das Innenbauteil 50 aufgeschoben und ist formschlüssig am Innenbauteil 50 befestigt, wie im Weiteren noch erläutert wird.

Die Betätigungshandhabe 30 des Spenders 100 ist in Richtung des Pfeils 6 aufgrund der Verformbarkeit der Kunststoffbrücke 28 verschwenkbar, so dass der Nasalapplikator 12 und der Stößel 40 mit verschwenkt werden. Durch das Verschwenken des Stößels 40 wird das Auslassventil 112 geöffnet und Flüssigkeit strömt durch den Innenkanal 90 des Stößels 40 nach oben, gelangt dann in einen Zwischenraum 48 zwischen dem Zentralstift 52 und dem Hülsenabschnitt 56 des Innenbauteils 50 des Nasalapplikators 12 und wird von dort aus durch einen gemeinsam vom Innenbauteil 50 und dem Außenbauteil 60 definierten Applikatorkanal 92 bis zur Austragöffnung 98 gefördert, von wo die Flüssigkeit ausgetragen werden kann. Zur Abdichtung der Bauteile 50, 60 gegeneinander weist das Außenbauteil eine Hülse 66 auf, deren Außenseite flüssigkeitsdicht an der innenseitigen Dichtfläche 56A der Hülse 56 anliegt.

Der Austragöffnung 98 ist eine Wirbelkammer 96 vorgeschaltet, deren außenbauteilseitige Wandung in Fig. 2 näher dargestellt ist. Die Wirbelkammer 96 ist durch das Außenbauteil 60 und das Innenbauteil 50, nämlich die Stirnseite des Zentralstiftes 52, gemeinsam begrenzt. Sie weist schräggestellte Kanäle 96A auf, durch die Flüssigkeit drallbehaftet in die Wirbelkammer 96 einströmen kann. Dieser Drall bewirkt beim Austreten durch die Austragöffnung 98 die Bildung eines kegelförmigen Sprühstrahls.

Das Außenbauteil 60 ist im Falle der Gestaltung der Fig. 1A und 1B sowie auch bei beiden Gestaltungen gemäß Fig. 3 und 4 als begrenzt relativbewegliches Außenbauteil 60 ausgebildet.

Bezug nehmend auf die Fig. 3 sowie 3A und 3B soll dies erläutert werden. Aus der Fig. 3 ist diese erste Ausgestaltung eines Austragkopfes in einer perspektivischen Darstellung zu sehen. Hieraus ist ersichtlich, dass die Basis 20 und die Betätigungshandhabe 30 mit der Betätigungsfläche 32 zwar einstückig miteinander verbunden sind, durch einen insgesamt etwa 300° überspannenden Spalt 26 jedoch ausreichend voneinander getrennt sind, um nach dem Brechen einer Sicherungsbrücke 29 eine Relativbeweglichkeit zu gestatten.

Das Außenbauteil 60 ist bei dieser Gestaltung als überlagert rotativ und linear gegenüber dem Innenbauteil 50 und der Betätigungshandhabe 30 ausgebildet. Bezug nehmend auf die Fig. 3A und 3B wird dies dadurch erzielt, dass an der Innenseite einer Mantelfläche 68 des Außenbauteils 60 sowie einer Außenfläche am Hülsenabschnitt 56 eine Gewindeverbindung geschaffen ist. An der Außenseite des Hülsenabschnitts ist hierfür eine gewindeartige Führungsstruktur 56B vorgesehen. Durch Drehen des Außenbauteils 60 kann somit bezogen auf die in Fig. 3A dargestellte Richtung 2 eine Verlagerung des Außenbauteils 60 gegenüber dem Innenbauteil 50 erreicht werden.

Dabei sind durch nicht dargestellte Anschläge das Innenbauteil 50 und das Außenbauteil 60 dafür ausgebildet, zwischen zwei um 180° voneinander beabstandete Endlagen gegeneinander verdreht zu werden, wobei dies eine axiale Verlagerung des Außenbauteils um wenige Millimeter bewirkt.

Die erste der beiden Endlagen, die in Fig. 3A dargestellt ist, ist dafür ausgebildet, einen Sprühstrahl zu ermöglichen, also eine Zerstäubung der Flüssigkeit beim Austrag. Flüssigkeit, die bei Betätigung in den Applikatorkanal 92 einströmt, muss in dieser Relativstellung durch die aus Fig. 2 ersichtlichen Wirbelkanäle 96A in die Wirbelkammer 96 einströmen, so dass der gesamte Flüssigkeitsstrom mit einem Drall versehen wird und als konischer Sprühstrahl austritt.

In der zweiten Endlage der Fig. 3B ist die Austragöffnung 98 ebenso wie der die Wirbelkanäle 96A aufweisende Steg 96B vom distalen Ende des Zentralstiftes 52 beabstandet, so dass nur ein geringer Anteil der Flüssigkeit, die in Richtung der Austragöffnung 98 strömt, mit einem Drall versehen wird. Der Austrag erfolgt deswegen in Form eines weitgehend oder vollständig unzerstäubten Strahls (Jet).

Durch die Beabstandung der Endlagen um 180° wird erzielt, dass trotz einer nicht rotationssymmetrischen Formgebung des Außenbauteils 60, die aus Fig. 3 zu ersehen ist, das subjektive Empfinden beim Einschieben des Nasalapplikators 12 in das Nasenloch unabhängig von der gerade erzielten Endstellung identisch bleibt. Dies wird durch die im Wesentlichen ebenensymmetrische Außenform des Außenbauteils 60 erzielt.

Wie insbesondere anhand der Fig. 3A und 3B ersichtlich ist, ist zwischen der Betätigungsfläche 32 und dem Innenbauteil 50 eine nutartige Vertiefung 38 vorgesehen, in die ein proximaler Rand 62 des Außenbauteils 60 hineinragt. Wie anhand des Vergleiches der Fig. 3A und 3B ersichtlich ist, ragt der proximale Rand 62 soweit in die Vertiefung 38 hinein, dass er unabhängig von der axialen Verlagerung des Außenbauteils 60 stets innerhalb dieser Vertiefung 38 verbleibt. Da nur ein geringer Spalt zwischen der Außenseite des Außenbauteils 60 und der äußeren Flanke der Vertiefung 38 verbleibt, ist das Eindringen von Schmutz hier unwahrscheinlich. Zudem wird eine ästhetisch vorteilhafte Wahrnehmung erzielt, weil der proximale Rand 62 des Außenbauteils für den Endbenutzer in der Regel nicht erkennbar ist.

Bei der Ausgestaltung gemäß der Figuren 4, 4A und 4B liegt der wesentliche Unterschied zur vorangegangenen Ausführungsform darin, dass hier keine rotative Relativverlagerung des Außenbauteils 60 gegenüber dem Innenbauteil 50 vorgesehen ist. Vielmehr weist das Außenbauteil 60 eine im Querschnitt eher elliptische Formgebung auf, die zusammenwirkend mit der nutartigen Vertiefung 38 ein Verdrehen verhindert.

Dennoch ist wiederum eine Relativverlagerbarkeit des Außenbauteils 60 gegenüber dem Innenbauteil 50 vorgesehen, nämlich eine rein lineare Beweglichkeit. Der Benutzer kann das Außenbauteil 60 in Richtung der Betätigungshandhabe 30 drücken und dabei den Zustand der Fig. 4A erzielen, bei dem wiederum ähnlich dem Zustand der Fig. 3A erzwungen wird, dass die aus dem Applikatorkanal 92 zuströmende Flüssigkeit durch die Wirbelkanäle 96A in die Wirbelkammer 96 gelangen und somit in Form eines Sprühstrahls austreten.

Wird hingegen das Außenbauteil 60 nach oben verlagert, also von der Betätigungshandhabe 30 entfernt, so stellt sich der Zustand der Fig. 4B ein, der wiederum eher die Erzeugung eines Jets, also eines unzerstäubten Strahls, bewirkt.

Das Innenbauteil 50 ist bei der Ausgestaltung der Fig. 4A und 4B sehr ähnlich dem der Fig. 3A und 3B ausgestaltet. Allerdings ist an der Außenseite der Hülse 56 eine Raststruktur 56A vorgesehen, die ein Verrasten des Außenbauteils 60 in der Position der Fig. 4A gestatten. Auf diese Art und Weise wird verhindert, dass das Außenbauteil 60 infolge des anliegenden Flüssigkeitsdrucks selbsttätig und ungewollt in die Stellung der Fig. 4B übergeht.

## Patentansprüche

1. Austragkopf (10) für die nasale Applikation von pharmazeutischen Flüssigkeit aus einem Druckspeicher (110), welcher über ein Auslassventil (112) mit einem Ventilstutzen (114) verfügt, der gegen eine Federkraft zum Öffnen des Auslassventils (112) kraftbeaufschlagbar ist, mit den folgenden Merkmalen:
a. der Austragkopf (10) verfügt über eine Basis (20) und eine daran vorgesehene Kopplungseinrichtung (22), mittels derer der Austragkopf (10) am Druckspeicher (110) befestigbar ist, und
b. der Austragkopf (10) verfügt über eine verlagerbare Betätigungsfläche (32), die gegenüber der Basis (20) verlagerbar ist, und an der ein als Hohlrohr (42) ausgestalteter Stößel (40) vorgesehen ist, der zum Zwecke der Kraftbeaufschlagung des Ventilstutzens (114) des Druckspeichers (110) gemeinsam mit der Betätigungsfläche (32) verlagerbar ist,
c. der Austragkopf (10) verfügt über einen Nasalapplikator (12), der sich von der Betätigungsfläche (32) aus nach außen erstreckt und an dessen Ende eine Austragöffnung (98) vorgesehen ist, die durch einen Applikatorkanal (92) des Nasalapplikators (12) hindurch mit dem Hohlrohr (42) fluidkommunizierend verbunden ist,
**gekennzeichnet durch** die folgenden zusätzlichen Merkmale:
d. der Nasalapplikator (12) verfügt über ein Innenbauteil (50), welches einstückig mit der Betätigungsfläche (32) verbunden ist, und
e. der Nasalapplikator (12) verfügt über ein Außenbauteil (60), welches als vom Innenbauteil (50) getrenntes Bauteil ausgebildet ist und das Innenbauteil (50) umgebend an diesem angebracht ist, und
f. die Austragöffnung (98) ist als Durchbrechung des Außenbauteils (60) vorgesehen, und
g. das Außenbauteil (60) und das Innenbauteil (50) begrenzen gemeinsam den Applikatorkanal (92).

2. Austragkopf (10) nach Anspruch 1 mit den folgenden zusätzlichen Merkmalen:
a. das Innenbauteil (50) und das Außenbauteil (60) bilden gemeinsam eine der Austragöffnung vorgelagerte Wirbelkammer (96).

3. Austragkopf (10) nach Anspruch 1 oder 2 mit den folgenden zusätzlichen Merkmalen:
a. das Außenbauteil (60) ist derart am Innenbauteil (50) angebracht, dass das Außenbauteil (60) im angebrachten Zustand gegenüber dem Innenbauteil (50) relativbeweglich ist, und
b. das Innenbauteil (50) und das Außenbauteil (60) sind derart ausgebildet, dass durch eine Relativverlagerung des Innenbauteils (50) und des Außenbauteils (60) der Applikatorkanal (92) und/oder die Wirbelkammer (96) geometrisch veränderlich ist.

4. Austragkopf (10) nach Anspruch 3 mit den folgenden zusätzlichen Merkmalen:
a. das Innenbauteil (50) und das Außenbauteil (60) sind derart ausgebildet, dass durch eine Relativverlagerung des Innenbauteils (50) und des Außenbauteils (60) der Applikatorkanal (92) bezüglich seines lichten Querschnitts veränderlich ist,
insbesondere mit dem zusätzlichen Merkmal:
b. das Innenbauteil (50) und das Außenbauteil (60) sind derart ausgebildet, dass durch eine Relativverlagerung des Innenbauteils (50) und des Außenbauteils (60) der Applikatorkanal (92) verschließbar ist.

5. Austragkopf (10) nach Anspruch 3 oder 4 mit den folgenden zusätzlichen Merkmalen:
a. das Innenbauteil (50) und das Außenbauteil (60) sind derart ausgebildet, dass durch die Relativbeweglichkeit des Innenbauteils (50) und des Außenbauteils (60) bei gleichem Einströmdruck von Flüssigkeit in den Nasalapplikator (12) die Charakteristik der durch die Austragöffnung (98) ausströmenden Flüssigkeit beeinflussbar ist,
insbesondere mit mindestens einem der folgenden zusätzlichen Merkmale:
b. in zwei unterschiedlichen Stellungen des Außenbauteils (60) relativ zum Innenbauteil (50) werden bei gleichem Einströmdruck unterschiedliche Flüssigkeitsströme durch die Austragöffnung (98) ausgegeben, und/oder
c. in zwei unterschiedlichen Stellungen des Außenbauteils (60) relativ zum Innenbauteil (50) wird bei gleichem Einströmdruck Flüssigkeit mit unterschiedlichem Maß von Tröpfchenbildung oder unterschiedlicher Tröpfchengröße ausgegeben, und/oder
d. in zwei unterschiedlichen Stellungen des Außenbauteils (60) relativ zum Innenbauteil (50) wird in unterschiedlichem Maße ein Drall in die auszugebende Flüssigkeit induziert.

6. Austragkopf (10) nach einem der Ansprüche 3 bis 5 mit einem der folgenden zusätzlichen Merkmale:
a. das Außenbauteil (60) ist gegenüber dem Innenbauteil (50) rein linear beweglich, oder
b. das Außenbauteil ist gegenüber dem Innenbauteil rein linear rotativ, oder
c. das Außenbauteil (60) ist gegenüber dem Innenbauteil (50) überlagert linear und rotativ beweglich.

7. Austragkopf (10) nach einem der vorstehenden Ansprüche mit den folgenden zusätzlichen Merkmalen:
a. in einem Übergangsbereich zwischen dem Innenbauteil (50) und der Betätigungsfläche (32) ist eine Vertiefung (38) vorgesehen, und
b. ein proximaler Rand (62) des Außenbauteils (60) taucht in mindestens einer Endstellung des Außenbauteils (60) gegenüber dem Innenbauteil (50) in diese Vertiefung (38) ein.

8. Austragkopf (10) nach Anspruch 7 mit dem folgenden zusätzlichen Merkmal:
a. das Innenbauteil (50) und das Außenbauteil (60) sind zwischen zwei Endstellungen gegeneinander beweglich, wobei der proximale Rand (62) des Außenbauteils zumindest abschnittsweise und vorzugsweise umlaufend in beiden Endstellungen innerhalb derVertiefung (38) angeordnet ist.

9. Austragkopf (10) nach einem der vorstehenden Ansprüche mit den folgenden zusätzlichen Merkmalen:
a. das Innenbauteil (50) verfügt über einen ersten Hülsenabschnitt (56), und
b. das Außenbauteil (60) verfügt über einen zweiten Hülsenabschnitt (66), und
c. eine Außenseite des ersten Hülsenabschnitts (56) weist Fixierungsmittel und/oder Führungsmittel (56A) auf, mittels derer das Außenbauteil (60) am Innenbauteil (50) befestigt und/oder geführt ist, und
d. eine Innenseite des ersten Hülsenabschnitts (56) weist eine Dichtfläche (56B) auf, an der der zweite Hülsenabschnitt (66) derart dichtend anliegt, dass ein gemeinsamer Innenraum (93) beider Hülsenabschnitte einen Abschnitt des Applikatorkanals (92) bildet, mittels dessen die Austragöffnung (98) mit dem als Hohlrohr (42) ausgebildeten Stößel (40) verbunden ist.

10. Austragkopf (10) nach einem der vorstehenden Ansprüche mit mindestens einem der folgenden Merkmale:
a. die Betätigungsfläche (32) und die Basis (20) und die daran vorgesehene Kopplungseinrichtung (22) des Austragkopfes (10) sind als einstückiges Bauteil ausgebildet und/oder
b. die Betätigungsfläche (32) und der als Hohlrohr (42) ausgebildete Stößel (40) sind als einstückiges Bauteil ausgebildet.

11. Austragkopf (10) nach einem der vorstehenden Ansprüche mit folgendem Merkmal:
a. das Außenbauteil (60) und die Betätigungsfläche (32) weisen unterschiedliche Farbgebungen und/oder unterschiedliche Materialen auf.

12. Austragkopf (10) nach einem der vorstehenden Ansprüche mit folgendem Merkmal:
a. die Betätigungsfläche (32) ist schwenkbeweglich an der Basis (20) angebracht, vorzugsweise durch eine einstückig die Basis (20) und die Betätigungsfläche (32) verbindende Kunststoffbrücke (28), die als Schwenkgelenk agiert,
vorzugsweise mit dem zusätzlichen Merkmal:
b. auf einer der Kunststoffbrücke (28) gegenüberliegenden Seite ist im Lieferzustand eine Sicherungsbrücke (29) vorgesehen, die die Betätigungsfläche (32) und die Basis (20) einstückig verbindet und bei Erstbetätigung bestimmungsgemäß bricht.

13. Austragkopf (10) nach einem der vorstehenden Ansprüche mit mindestens einem der folgenden Merkmale:
a. eine durch die Austragrichtung (2) der Austragöffnung (98) und/oder durch eine die Rotationsmittelachse des Außenbauteils (60) gegenüber dem Innenbauteil (50) definierte Achse schließt mit einer Aufsetzrichtung (4) des Austragkopfs auf einen Druckspeicher (110) einen Winkel α zwischen 5° und 85° ein, vorzugsweise einen Winkel zwischen 10° und 50°, und/oder
b. der Nasalapplikator (12) ist als länglicher Applikator mit einer frei von der Betätigungsfläche (32) hervorragenden Länge von mindestens 20mm ausgebildet, und/oder
c. der Nasalapplikator (12) ist als länglicher Applikator mit einer sich in Richtung der Austragöffnung (98) verjüngenden Formgebung ausgebildet, und/oder
d. die Austragöffnung (98) weist eine minimale Querschnittsfläche von weniger als 4 mm2 auf, insbesondere von weniger als 2 mm2, und/oder
e. der Wirbelkammer (96) ist mindestens eine Leitfläche (96A) zur Erzeugung eines Dralls zugeordnet, die gegenüber einer Tangentialrichtung zur Austragrichtung (2) schräggestellt ist, wobei diese Leitfläche (96A) an einer Innenseite des Außenbauteils (60) vorgesehen ist, und/oder
f. die Kopplungseinrichtung (22) umfasst einstückig an der Basis angeformte Rastkanten (24), die auf einer Kopplungskante des Druckspeichers (110) aufgerastet werden, und/oder
g. das Innenbauteil (50) umfasst einen Hülsenabschnitt (56) zum Zwecke der Abdichtung gegenüber dem Außenbauteil (60) und/oder zum Zwecke der Kopplung des Außenbauteils (60) am Innenbauteil (50) sowie einen zentrischen Stift (52), dessen distales Ende (54) mit der Austragöffnung (98) zur Beeinflussung des Austrags zusammenwirkt, wobei ein durch das Hohlrohr (42) gebildeter Kanal (90) in einen Zwischenraum (58) zwischen dem zentrischen Stift (52) und dem Hülsenabschnitt (56) mündet, und/oder
h. das Außenbauteil (60) ist durch Anschlagsflächen davon abgehalten, vom Innenbauteil trennbar zu sein, wobei diese Anschlagsflächen derart angeordnet und ausgebildet sind, dass sie durch eine erhöhte Kraftbeaufschlagung die Trennung des Außenbauteils vom Innenbauteil zerstörungsfrei gestatten, so dass das Außenbauteil gegen eine anderes Außenbauteil austauschbar ist.

14. Spender (100) zum Austrag pharmazeutischer Flüssigkeiten mit den folgenden Merkmalen:
a. der Spender (100) verfügt über einen Druckspeicher (110), in welchem pharmazeutische Flüssigkeit unter Druck gelagert ist und der seinerseits über eine Auslassventil (112) verfügt, und
b. das Auslassventil (112) verfügt über einen Ventilstutzen (114), der gegen eine Federkraft zum Öffnen des Ventils kraftbeaufschlagbar ist, und
b. der Spender (100) verfügt über einen Austragkopf (10) zur Ankoppelung an den Druckspeicher (110),
**gekennzeichnet durch** das Merkmal:
c. der Austragkopf (10) ist nach einem der vorstehenden Ansprüche ausgebildet.

## Claims

1. Discharge head (10) for nasal application of pharmaceutical liquid from a pressure accumulator (110) which has an outlet valve (112) with a valve stem (114) that can be subjected to force counter to a spring force in order to open the outlet valve (112), with the following features:
a. the discharge head (10) has a base (20) and, provided thereon, a coupling mechanism (22) by means of which the discharge head (10) can be secured on the pressure accumulator (110), and
b. the discharge head (10) has a displaceable actuating surface (32), which is displaceable with respect to the base (20) and on which a tappet (40) configured as a hollow tube (42) is provided, said tappet (40) being displaceable together with the actuating surface (32) for the purpose of applying force to the valve stem (114) of the pressure accumulator (110),
c. the discharge head (10) has a nasal applicator (12) which extends outwards from the actuating surface (32) and at the end of which a discharge opening (98) is provided which, by way of an applicator channel (92) of the nasal applicator (12), is connected in fluidic communication with the hollow tube (42),
**characterized by** the following additional features:
d. the nasal applicator (12) has an inner structural part (50), which is integrally connected to the actuating surface (32), and
e. the nasal applicator (12) has an outer structural part (60) which is configured as a structural part separate from the inner structural part (50) and which is mounted on and encloses the inner structural part (50), and
f. the discharge opening (98) is provided as an aperture of the outer structural part (60), and
g. the outer structural part (60) and the inner structural part (50) together delimit the applicator channel (92).

2. Discharge head (10) according to Claim 1, with the following additional features:
a. the inner structural part (50) and the outer structural part (60) together form a vortex chamber (96) before the discharge opening.

3. Discharge head (10) according to Claim 1 or 2, with the following additional features:
a. the outer structural part (60) is fitted on the inner structural part (50) in such a way that the outer structural part (60), in the fitted state, is movable relative to the inner structural part (50), and
b. the inner structural part (50) and the outer structural part (60) are configured in such a way that the geometry of the applicator channel (92) and/or of the vortex chamber (96) is modifiable by a relative displacement of the inner structural part (50) and of the outer structural part (60).

4. Discharge head (10) according to Claim 3, with the following additional features:
a. the inner structural part (50) and the outer structural part (60) are configured in such a way that, by a relative displacement of the inner structural part (50) and of the outer structural part (60), the applicator channel (92) is modifiable in terms of its clear cross section, in particular with the additional feature:
b. the inner structural part (50) and the outer structural part (60) are configured in such a way that, by a relative displacement of the inner structural part (50) and of the outer structural part (60), the applicator channel (92) is closable.

5. Discharge head (10) according to Claim 3 or 4, with the following additional features:
a. the inner structural part (50) and the outer structural part (60) are configured in such a way that, at the same inflow pressure of liquid into the nasal applicator (12), the characteristics of the liquid flowing out through the discharge opening (98) can be influenced by the relative mobility of the inner structural part (50) and of the outer structural part (60),
in particular with at least one of the following additional features:
b. at the same inflow pressure, different flows of liquid are dispensed through the discharge opening (98) in two different positions of the outer structural part (60) relative to the inner structural part (50), and/or
c. at the same inflow pressure, liquid with different degrees of droplet formation or with a different droplet size are dispensed in two different positions of the outer structural part (60) relative to the inner structural part (50), and/or
d. in two different positions of the outer structural part (60) relative to the inner structural part (50), swirling is induced to different extents in the liquid that is to be dispensed.

6. Discharge head (10) according to one of Claims 3 to 5, with one of the following additional features:
a. the outer structural part (60) is movable purely linearly with respect to the inner structural part (50), or
b. the outer structural part is purely linearly rotatably with respect to the inner structural part, or
c. the outer structural part (60) is movable with respect to the inner structural part (50) in a superposed linear and rotational movement.

7. Discharge head (10) according to one of the preceding claims, with the following additional features:
a. a depression (38) is provided in a transition region between the inner structural part (50) and the actuating surface (32), and
b. a proximal edge (62) of the outer structural part (60) engages in this depression (38) in at least one end position of the outer structural part (60) with respect to the inner structural part (50) .

8. Discharge head (10) according to Claim 7, with the following additional feature:
a. the inner structural part (50) and the outer structural part (60) are movable relative to each other between two end positions, wherein the proximal edge (62) of the outer structural part is arranged at least in some regions, and preferably circumferentially, inside the depression (38) in both end positions.

9. Discharge head (10) according to one of the preceding claims, with the following additional features:
a. the inner structural part (50) has a first sleeve portion (56), and
b. the outer structural part (60) has a second sleeve portion (66), and
c. an outer face of the first sleeve portion (56) has fixing means and/or guiding means (56A), by means of which the outer structural part (60) is secured and/or guided on the inner structural part (50), and
d. an inner face of the first sleeve portion (56) has a sealing surface (56B) on which the second sleeve portion (66) bears sealingly in such a way that a common interior (93) of both sleeve portions forms a portion of the applicator channel (92), by means of which the discharge opening (98) is connected to the tappet (40) configured as a hollow tube (42).

10. Discharge head (10) according to one of the preceding claims, with at least one of the following features:
a. the actuating surface (32) and the base (20), and the coupling mechanism (22) of the discharge head (10) provided thereon, are configured as a one-piece structural part, and/or
b. the actuating surface (32) and the tappet (40), configured as a hollow tube (42), are configured as a one-piece structural part.

11. Discharge head (10) according to one of the preceding claims, with the following feature:
a. the outer structural part (60) and the actuating surface (32) have different colours and/or different materials.

12. Discharge head (10) according to one of the preceding claims, with the additional feature:
a. the actuating surface (32) is mounted on the base (20) so as to be pivotable, preferably by means of a plastic bridge (28) which integrally connects the base (20) and the actuating surface (32) and which acts as a pivot hinge,
preferably with the additional feature:
b. a safety bridge (29) is provided on a side opposite the plastic bridge (28) in the delivery state, which safety bridge (29) integrally connects the actuating surface (32) and the base (20) and intentionally breaks at the time of the first actuation.

13. Discharge head (10) according to one of the preceding claims, with at least one of the following features:
a. an axis defined by the discharge direction (2) of the discharge opening (98) and/or by a central axis of rotation of the outer structural part (60) with respect to the inner structural part (50) encloses, with a direction of fitting (4) of the discharge head onto a pressure accumulator (110), an angle α of between 5° and 85°, preferably an angle of between 10° and 50°, and/or
b. the nasal applicator (12) is configured as an elongate applicator with a length of at least 20 mm protruding freely from the actuating surface (32), and/or
c. the nasal applicator (12) is configured as an elongate applicator with a shape that tapers in the direction of the discharge opening (98), and/or
d. the discharge opening (98) has a minimum cross-sectional area of less than 4 mm², in particular of less than 2 mm², and/or
e. the vortex chamber (96) is assigned at least one guide surface (96A) for generating a swirling motion, which guide surface (96A) is inclined with respect to a tangential direction to the discharge direction (2), wherein this guide surface (96A) is provided on an inner face of the outer structural part (60), and/or
f. the coupling mechanism (22) comprises latching edges (24) which are formed integrally on the base and which are latched onto a coupling edge of the pressure accumulator (110), and/or
g. the inner structural part (50) comprises a sleeve portion (56) for the purpose of sealing with respect to the outer structural part (60) and/or for the purpose of coupling the outer structural part (60) to the inner structural part (50), and a central pin (52), of which the distal end (54) cooperates with the discharge opening (98) to influence the discharge, wherein a channel (90) formed by the hollow tube (42) opens into a space (58) between the central pin (52) and the sleeve portion (56), and/or
h. the outer structural part (60) is prevented by stop surfaces from being separable from the inner structural part, wherein these stop surfaces are arranged and configured in such a way that, when increased force is applied, they allow the outer structural part to be separated from the inner structural part without destruction, such that the outer structural part can be replaced by another outer structural part.

14. Dispenser (100) for discharging pharmaceutical liquids, with the following features:
a. the dispenser (100) has a pressure accumulator (110) in which pharmaceutical liquid is stored under pressure and which in turn has an outlet valve (112), and
b. the outlet valve (112) has a valve stem (114) which can be subjected to force counter to a spring force in order to open the valve, and
c. the dispenser (100) has a discharge head (10) for coupling to the pressure accumulator (110),
**characterized by** the feature:
d. the discharge head (10) is configured according to one of the preceding claims.

## Revendications

1. Tête de distribution (10) pour l'application nasale de liquide pharmaceutique à partir d'un récipient sous pression (110), qui dispose d'une valve de sortie (112) avec un embout de valve (114) qui peut être sollicité par force à l'encontre d'une force de ressort pour ouvrir la valve de sortie (112), comprenant les caractéristiques suivantes :
a. la tête de distribution (10) dispose d'une base (20) et d'un dispositif d'accouplement (22) prévu sur celle-ci au moyen duquel la tête de distribution (10) peut être fixée sur le récipient sous pression (110), et
b. la tête de distribution (10) dispose d'une surface d'actionnement déplaçable (32) qui peut être déplacée par rapport à la base (20), et au niveau de laquelle est prévu un poussoir (40) configuré sous forme de tube creux (42) qui peut être déplacé conjointement avec la surface d'actionnement (32) afin de solliciter par force l'embout de valve (114) du récipient sous pression (110),
c. la tête de distribution (10) dispose d'un applicateur nasal (12) qui s'étend vers l'extérieur depuis la surface d'actionnement (32) et à l'extrémité duquel est prévue une ouverture de distribution (98) qui est connectée fluidiquement au tube creux (42) à travers un canal d'applicateur (92) de l'applicateur nasal (12),
**caractérisée par** les caractéristiques supplémentaires suivantes :
d. l'applicateur nasal (12) dispose d'un composant intérieur (50) qui est connecté d'une seule pièce à la surface d'actionnement (32), et
e. l'applicateur nasal (12) dispose d'un composant extérieur (60) qui est réalisé sous forme de composant séparé du composant intérieur (50) et qui est monté sur le composant intérieur (50) en entourant celui-ci, et
f. l'ouverture de distribution (98) est prévue sous forme d'orifice du composant extérieur (60), et
g. le composant extérieur (60) et le composant intérieur (50) délimitent ensemble le canal d'applicateur (92).

2. Tête de distribution (10) selon la revendication 1, comprenant la caractéristique supplémentaire suivante :
a. le composant intérieur (50) et le composant extérieur (60) forment ensemble une chambre de tourbillonnement (96) disposée en amont de l'ouverture de distribution.

3. Tête de distribution (10) selon la revendication 1 ou 2, comprenant les caractéristiques supplémentaires suivantes :
a. le composant extérieur (60) est monté sur le composant intérieur (50) de telle sorte que le composant extérieur (60), dans l'état monté, puisse être déplacé par rapport au composant intérieur (50), et
b. le composant intérieur (50) et le composant extérieur (60) sont réalisés de telle sorte que par déplacement relatif du composant intérieur (50) et du composant extérieur (60), la géométrie du canal d'applicateur (92) et/ou de la chambre de tourbillonnement (96) puisse être variée.

4. Tête de distribution (10) selon la revendication 3, comprenant les caractéristiques supplémentaires suivantes :
a. le composant intérieur (50) et le composant extérieur (60) sont réalisés de telle sorte que par déplacement relatif du composant intérieur (50) et du composant extérieur (60), la section transversale intérieure du canal d'applicateur (92) puisse être variée,
en particulier comprenant la caractéristique supplémentaire suivante :
b. le composant intérieur (50) et le composant extérieur (60) sont réalisés de telle sorte que par déplacement relatif du composant intérieur (50) et du composant extérieur (60), le canal d'applicateur (92) puisse être fermé.

5. Tête de distribution (10) selon la revendication 3 ou 4, comprenant les caractéristiques supplémentaires suivantes :
a. le composant intérieur (50) et le composant extérieur (60) sont réalisés de telle sorte que par déplacement relatif du composant intérieur (50) et du composant extérieur (60) pour une pression d'afflux identique de liquide dans l'applicateur nasal (12), la caractéristique du liquide sortant à travers l'ouverture de distribution (98) puissent être influencée,
en particulier comprenant au moins l'une des caractéristiques supplémentaires suivantes :
b. dans deux positions différentes du composant extérieur (60) par rapport au composant intérieur (50), pour une même pression d'afflux, différents flux de liquide sont distribués à travers l'ouverture de distribution (98), et/ou
c. dans deux positions différentes du composant extérieur (60) par rapport au composant intérieur (50), pour une même pression d'afflux, du liquide est distribué avec une formation de gouttelettes ou une taille de gouttelettes différentes dans une mesure différente, et/ou
d. dans deux positions différentes du composant extérieur (60) par rapport au composant intérieur (50), un tourbillon est induit dans une mesure différente dans le liquide à distribuer.

6. Tête de distribution (10) selon l'une quelconque des revendications 3 à 5, comprenant l'une des caractéristiques supplémentaires suivantes :
a. le composant extérieur (60) est déplaçable purement linéairement par rapport au composant intérieur (50), ou
b. le composant extérieur est purement de manière rotative linéairement par rapport au composant intérieur, ou
c. le composant extérieur (60) est déplaçable de manière superposée linéairement et de manière rotative par rapport au composant intérieur (50) .

7. Tête de distribution (10) selon l'une quelconque des revendications précédentes, comprenant les caractéristiques supplémentaires suivantes :
a. dans une région de transition entre le composant intérieur (50) et la surface d'actionnement (32) est prévu un renfoncement (38), et
b. un bord proximal (62) du composant extérieur (60), dans au moins une position d'extrémité du composant extérieur (60) par rapport au composant intérieur (50), s'enfonce dans ce renfoncement (38).

8. Tête de distribution (10) selon la revendication 7, comprenant la caractéristique supplémentaire suivante :
a. le composant intérieur (50) et le composant extérieur (60) peuvent être déplacés l'un par rapport à l'autre entre deux positions d'extrémité, le bord proximal (62) du composant extérieur étant disposé à l'intérieur du renfoncement (38) au moins en partie et de préférence circonférentiellement dans les deux positions d'extrémité.

9. Tête de distribution (10) selon l'une quelconque des revendications précédentes, comprenant les caractéristiques supplémentaires suivantes :
a. le composant intérieur (50) dispose d'une première partie de douille (56), et
b. le composant extérieur (60) dispose d'une deuxième partie de douille (66), et
c. un côté extérieur de la première partie de douille (56) présente des moyens de fixation et/ou des moyens de guidage (56A), au moyen desquels le composant extérieur (60) est fixé et/ou est guidé sur le composant intérieur (50), et
d. un côté intérieur de la première partie de douille (56) présente une surface d'étanchéité (56B) contre laquelle la deuxième partie de douille (66) s'applique de manière hermétique de telle sorte qu'un espace intérieur commun (93) des deux parties de douille forme une partie du canal d'applicateur (92) au moyen de laquelle l'ouverture de distribution (98) est connectée au poussoir (40) réalisé sous forme de tube creux (42).

10. Tête de distribution (10) selon l'une quelconque des revendications précédentes, comprenant au moins l'une des caractéristiques suivantes :
a. la surface d'actionnement (32) et la base (20) et le dispositif d'accouplement (22) de la tête de distribution (10) prévu sur celle-ci sont réalisés sous forme de composant d'une seule pièce et/ou
b. la surface d'actionnement (32) et le poussoir (40) réalisé sous forme de tube creux (42) sont réalisés sous forme de composant d'une seule pièce.

11. Tête de distribution (10) selon l'une quelconque des revendications précédentes, comprenant la caractéristique suivante :
a. le composant extérieur (60) et la surface d'actionnement (32) présentent des colorations différentes et/ou des matériaux différents.

12. Tête de distribution (10) selon l'une quelconque des revendications précédentes, comprenant la caractéristique suivante :
a. la surface d'actionnement (32) est montée de manière déplaçable par pivotement sur la base (20), de préférence par un pont en plastique (28) reliant d'une seule pièce la base (20) et la surface d'actionnement (32), lequel agit en tant qu'articulation pivotante,
de préférence comprenant la caractéristique suivante :
b. sur un côté opposé au pont en plastique (28), dans l'état livré, est prévu un pont de fixation (29) qui relie d'une seule pièce la surface d'actionnement (32) et la base (20) et qui se rompt de manière conforme aux spécifications lors du premier actionnement.

13. Tête de distribution (10) selon l'une quelconque des revendications précédentes, comprenant au moins l'une des caractéristiques suivantes :
a. un axe défini par la direction de distribution (2) de l'ouverture de distribution (98) et/ou par un axe médian de rotation du composant extérieur (60) par rapport au composant intérieur (50), forme avec une direction de pose (4) de la tête de distribution sur un récipient sous pression (110), un angle α compris entre 5° et 85°, de préférence un angle compris entre 10° et 50°, et/ou
b. l'applicateur nasal (12) est réalisé sous forme d'applicateur allongé avec une longueur d'au moins 20 mm dépassant librement de la surface d'actionnement (32), et/ou
c. l'applicateur nasal (12) est réalisé sous forme d'applicateur allongé avec une forme se rétrécissant dans la direction de l'ouverture de distribution (98), et/ou
d. l'ouverture de distribution (98) présente une surface minimale en section transversale inférieure à 4 mm², en particulier inférieure à 2 mm², et/ou
e. la chambre de tourbillonnement (96) est associée à au moins une surface directrice (96A) pour générer un tourbillon, laquelle est orientée obliquement par rapport à une direction tangentielle à la direction de distribution (2), cette surface directrice (96A) étant prévue au niveau d'un côté intérieur du composant extérieur (60), et/ou
f. le dispositif d'accouplement (22) comprend des arêtes d'encliquetage (24) façonnées d'une seule pièce sur la base, qui sont encliquetées sur une arête d'accouplement du récipient sous pression (110), et/ou
g. le composant intérieur (50) comprend une partie de douille (56) afin de réaliser l'étanchéité par rapport au composant extérieur (60) et/ou afin d'accoupler le composant extérieur (60) au composant intérieur (50), ainsi qu'une goupille centrale (52) dont l'extrémité distale (54) coopère avec l'ouverture de distribution (98) pour influencer la distribution, un canal (90) formé par le tube creux (42) débouchant dans un espace intermédiaire (58) entre la goupille centrale (52) et la partie de douille (56), et/ou
h. le composant extérieur (60) est empêché par des surfaces de butée, de se séparer du composant intérieur, ces surfaces de butée étant disposées et réalisées de telle sorte qu'elles permettent, par une sollicitation par force accrue, la séparation du composant extérieur du composant intérieur sans destruction, de telle sorte que le composant extérieur puisse être remplacé par un autre composant extérieur.

14. Distributeur (100) pour la distribution de liquides pharmaceutiques, comprenant les caractéristiques suivantes :
a. le distributeur (100) dispose d'un récipient sous pression (110) dans lequel est stocké un liquide pharmaceutique sous pression, et qui dispose pour sa part d'une valve de sortie (112), et
b. la valve de sortie (112) dispose d'un embout de valve (114) qui peut être sollicité par force à l'encontre d'une force de ressort pour ouvrir la valve, et
c. le distributeur (100) dispose d'une tête de distribution (10) pour l'accouplement au récipient sous pression (110),
**caractérisé par** la caractéristique suivante :
d. la tête de distribution (10) est réalisée selon l'une quelconque des revendications précédentes.
